# EUROPEAN PATENT APPLICATION

(11) **EP 3 029 151 A1**
(43) Date of publication of application: **08.06.2016**
(21) Application number: 14306942.5
(22) Date of filing: 03.12.2014
(51) Int. Cl.: C12Q 1/68, G01N 33/574

(54) **Use of a novel combination of blood biomarkers for malignant erythroderma**

(71) Applicant: INSTITUT NATIONAL DE LA SANTE ET DE LA RECHERCHE MEDICALE (INSERM), 75013 Paris (FR); Université Paris Diderot - Paris 7, 75013 Paris (FR); CENTRE NATIONAL DE LA RECHERCHE SCIENTIFIQUE -CNRS-, 75016 Paris (FR); ASSISTANCE PUBLIQUE, HOPITAUX DE PARIS, 75004 Paris (FR)
(72) Inventor: Michel, Laurence, 75017 Paris (FR); Bensussan, Armand, 75015 Paris (FR); Bagot, Martine, 75007 Paris (FR)
(74) Representative: Regimbeau

(57) **Abstract**

The present invention relates to methods and means for assessing risk, diagnosing, and prognosing malignant erythroderma in human subjects.

## Description

The present invention relates to methods and means for assessing risk, diagnosing, and prognosing malignant erythroderma in human subjects.

Erythroderma describe intense and widespread reddening of the skin due to either inflammatory or malignant skin diseases. Cutaneous T-cell lymphomas (CTCL) are a form of malignant erythroderma. CTCL form a rare heterogeneous group of non-Hodgkin lymphomas derived from skin-homing mature T-cells. The CTCL group comprises mycosis fungoides (MF), transformed mycosis fungoides, Sezary Syndrome (SS), Lymphomatoide Papulosis, and CD30+ cutaneous lymphomas. SS is an aggressive form of CTCL characterized by a clonal expansion of tumoral CD4⁺/CD45RO⁺ T cells and the appearance of these malignant T cells in the blood. MF and SS represent the most common subtypes of primary CTCL, with an incidence rate of 4.1/1,000,000 person-years and male predominance.

The biology of CTCL disease, and particularly SS, remains poorly understood. Current diagnosis of CTCL and particularly SS is based on cytological and histological observations of the presence or absence of tumoral cells in a sample collected from the suspected body area (observation of histopathological aspect on skin biopsies and/or presence of tumoral SS cells in the blood, via detection of cells showing a cerebriform nucleus). Such a diagnosis method is not fully reliable, notably at the early stages of the transformation of skin lymphocytes into malignant lymphocytes, when there are no tumoral cells in blood.

The differential diagnosis of erythroderma is by histology but is often difficult to interprete, even by a regular specialist. The main symptom is the appearance of generalised skin and swelling rednessinvolving 80% or more of the skin surface. This symptom is common to malignant erythroderma caused by CTCL and inflammatory erythroderma. As previously mentioned, the CTCL are a heterogeneous group of disorders from clinical, morphological, molecular, evolutionary, prognostic, and therapeutic points of view. In fact, patients are a very heterogeneous population; phenotypic biomarkers exist in CTCL tumor cells but their cutaneous expression can vary from one patient to another. Therefore, early diagnosis of malignant erythroderma is a real problem. Currently, methods of diagnosis involve analysis of skin biopsies, in combination with other batteries and optionally molecular phenotypic tests.

Today, there is a need for a diagnosis that is not only reliable and discriminant of CTCL, but also easy to make and of general application, regardless of the heterogeneity of the cutaneous biomarker profile of the patients. Similarly, there is a need for a suitable prognosis of CTCL.

The inventors provide a simple solution to make a diagnosis possible at a much earlier stage than what is done in the prior art. The prior art discloses biomarkers which can be used for diagnosis of erythroderma from skin biopsies. The use of the expression level of these markers is always combined with other means to check, confirm or refute the initial diagnosis. Accurate diagnosis is necessarily done by successive combinations of analyses.

Recently, the inventors have identified and used four blood biomarkers for the diagnosis of SS. The analysis of gene expression is by quantitative PCR (qRT-PCR). The quantification of the expression level of mRNA is via CD4⁺ T cells purified from SS patients. *(*Michel et al. Use of PLS3, Twist, CD158k/KIR3DL2, and NKp46 gene expression combination for reliable Sézary syndrome diagnosis. 2013*).* The TOX biomarker is not described in this combination of 4 biomarkers.

Particularly, the Tox marker is known in the MF wherein its expression level is analyzed via skin biopsies by histology *(*Zhang et al., Molecular biomarkers of early-stage Mycosis Fungoides, 2012*).* This paper shows that the analysis of the expression of RNA corresponding to the two biomarkers PDCD1 and TOX has a high discriminatory power between early mycosis fungoides (eMF) lesions and biopsies of dermatitis Benign. Furthermore, it is shown a high specific TOX staining cells in MF skin biopsies of patients with eMF. The results were analyzed by immunohistochemistry and immunofluorescence. The studies are done via skin biopsies. The ratio of the expression of TOX in biopsies eMF / NS is 10.38. This ratio is lower than the ratio that inventors obtain via an analysis of the level of expression of TOX in the blood for the diagnosis of SS. In the context of the invention, the ratio is about 86. Thus, this paper raises the issue of the lack of a standardized and reliable method for the diagnosis of MF.

However, beyond identification of specific biomarkers the expression of which may be linked to specific disease such as, SS MF, Lymphomatoide Papulosis or CD30+ cutaneous lymphomas, none of the publications cited above or known by the one skilled in the art, discloses a method for diagnosing malignant erythroderma, wherein the combination of five specific biomarkers allows easy diagnosis of malignant erythroderma in 100% of SS cases.

Consequently, there is still a need for more accurate and appropriate solutions for the diagnosis and therapy of diseases involving the proliferation of malignant T cells, such as malignant erythroderma versus inflammatory erythroderma, preferably CTCL-associated malignant erythroderma and more particularly SS.

Surprisingly, the present invention shows that a reliable diagnosis of a malignant erythroderma, preferably a CTCL, such as SS , MF, Lymphomatoide Papulosis or CD30+ cutaneous lymphomas, may be made by using specific combinations of biomarkers comprising, or consisting of, the biomarker Tox and at least one, two, three or four biomarkers selected from a restricted family of the following biomarkers PLS3, Twist, KIR3DL2, and NKp46.

The present inventors have found that, when combined, quantification of at least the biomarker Tox with another above-cited biomarker allows reliable and predictive diagnosis of a malignant erythroderma in a human subject.

More precisely, the inventors have found threshold expression level values for each of the five biomarkers Tox, PLS3, Twist, KIR3DL2 and NKp46 above, such that a diagnosis of a malignant erythroderma is possible.

Illustratively, it is shown in the examples herein that quantification of specific combinations of at least Tox and another biomarker selected among PLS3, Twist, KIR3DL2 and NKp46, allows a reliable prediction and diagnosis of a malignant erythroderma in a human subject. Illustratively, 100% of SS-positive individuals tested were correctly predicted using the combination of the five biomarkers.

The methods of the invention allow the accurate evaluation of risk for an individual's health due to malignant erythroderma at or before disease onset, thus reducing or minimizing the negative effects of malignant erythroderma-associated skin disease. In particular, the invention allows a better prediction of the risk of malignant erythroderma and, therefore, of subsequent complications. The methods of the invention can be applied in human subjects who are either free of clinical symptoms but with signs of erythroderma, or have elevated level or levels risk factors.

More precisely, by "CTCL" it is meant herein a CTCL-associated malignant erythroderma. More precisely the CTCL can be selected from the group consisting of MF, SS, Lymphomatoide Papulosis and CD30+ cutaneous lymphomas.

In the context of the invention, a "biomarker" is a skin protein marker indicative of malignant erythroderma in a human subject, that is to say a nucleic acid sequence which is specifically and significantly involved in malignant erythroderma onset. Preferably, a "biomarker" according to the invention is detected in the blood of the human subject.

The present invention relates to means for diagnosis, prognosis, treating and/or preventing malignant erythroderma, preferably CTCL in a human subject.

More precisely, the present invention provides means and methods for risk assessment and/or diagnosis and/or prognosis of malignant erythroderma and/or CTCL in humans, based on the detection of Tox and at least another biomarker selected among PLS3, Twist, KIR3DL2, and NKp46, in a whole blood sample.

An aspect of the invention concerns an *in vitro* method for risk assessment and/or diagnosis and/or prognosis of malignant erythroderma, preferably cutaneous t-cell lymphoma (CTCL), in a human subject, comprising a step of:
a) assessing the presence, in a blood sample of said subject, of the biomarker TOX and at least one biomarker selected from the group consisting of: Twist, PLS3, KIRD3DL2, and NKp46, thereby obtaining biomarker data from said subject; and
b) comparing the thus obtained biomarker data from said subject to biomarker data of reference obtained from healthy and/or diseased subjects, to make risk assessment and/or diagnosis and/or prognosis of malignant erythroderma, preferably CTCL, in said human subject.

By "risk assessment", it is meant herein that the present invention makes it possible to estimate or evaluate the risk of a human subject to develop malignant erythroderma (one skilled in the art could also say "predisposition or susceptibility assessment"). In this respect, an individual "at risk" of malignant erythroderma is an individual who has at least one risk factor known to contribute to the development of malignant erythroderma, including for instance:
- redskin
- swelling on skin surface.

By "data of reference", it is meant herein appropriate data that are obtained from control subjects who are healthy and/or diseased subjects, and that are used for qualitatively and/or quantitatively comparing to the data obtained from the subject under consideration. In particular, by "data of reference", it is meant herein the protein expression level and/or mRNA expression level obtained from one or more healthy subjects.

The prediction or risk generally implies that the risk is either increased or reduced.

As used herein, Tox, which encodes a nuclear protein of the high-mobility group family and is highly but transiently expressed in thymic tissue (Wilkinson et al., 2002), means an expression product of Human NKp46 gene and encompasses both the NKp46 protein expression product and the NKp46 mRNA expression product.

As used herein, PLS3, which may also be termed "T-plastin", means an expression product of Human Plastin 3 gene and encompasses both the PLS3 protein expression product and the PLS3 mRNA expression product.

As used herein, Twist, which may also be termed "Twist-related Protein 1 ", means an expression product of Human Twist gene (also termed "Twist1" gene) and encompasses both the Twist protein expression product and the Twist mRNA expression product.

As used herein, KIR3DL2, which may also be termed "CD158k", and which is a member of the "Killer immunoglobulin-like receptor" (KIR) family, means an expression product of KIR3DL2 gene and encompasses both the KIR3DL2 protein expression product and the KIR3DL2 mRNA expression product.

As used herein, NKp46, which may also be termed "CD335", which is NCR1 ("Natural Cytotoxicity Receptor 1 "), means an expression product of Human NKp46 gene and encompasses both the NKp46 protein expression product and the NKp46 mRNA expression product.

In the present specification, the name of each of the various bio markers of interest refers to the internationally recognized name of the corresponding gene, as found in internationally recognized gene sequences and protein sequences databases, including in the database from the HUGO Gene Nomenclature Committee, that is available from a website, in particular at the following Internet address : http://www.ene.ucl.ac.uk/nomenclature/index.html.

The terms "diseased subjects", "human subjects in need of a therapeutic treatment , "human subjects in need thereof" and "human subjects having malignant erythroderma" all refer to subjects suffering from malignant erythroderma, preferably from CTCL, more preferably from SS or MF. These terms can be used interchangeably. Preferably, the diseased subject did not receive any systemic treatment before diagnosis.

The terms "treatment" and "therapy" refer not only to ameliorating symptoms associated with malignant erythroderma, but also preventing or delaying the onset of the disease, and/or lessening the severity or frequency of symptoms of the malignant erythroderma.

According to various embodiments, the method described above is useful for:
- Identifying human subjects at risk for developing malignant Erythroderma, preferably a CTCL,
- Diagnosing malignant Erythroderma, preferably a CTCL in a human subject
- Selecting efficient and safe therapy to a human subject having malignant Erythroderma, preferably a CTCL
- Monitoring the effect of a therapy administered to a human subject having malignant Erythroderma, preferably a CTCL
- Predicting the effectiveness of a therapy to treat malignant Erythroderma, preferably a CTCL, in a human subject in need of such treatment
- Selecting efficient and safe preventing therapy to a human subject at risk for developing malignant Erythroderma, preferably a CTCL
- Monitoring the effect of a preventive therapy administered to a human subject at risk for developing malignant Erythroderma, preferably a CTCL
- Predicting the effectiveness of a therapy to prevent malignant Erythroderma, preferably a CTCL.

According to an embodiment of the *in vitro* method for diagnosing a malignant erythroderma as defined herein, a combination of the biomarker Tox and at least one biomarker selected among PLS3, Twist, KIR3DL2, and NKp46, is used.

According to another embodiment of the *in vitro* method for diagnosing a malignant erythroderma as defined herein, a combination of the biomarker Tox and at least two biomarkers selected among PLS3, Twist, KIR3DL2, and NKp46, is used.

According to yet another embodiment of the *in vitro* method for diagnosing a malignant erythroderma as defined herein, a combination of the biomarker Tox and at least three biomarkers selected among PLS3, Twist, KIR3DL2, and NKp46, is used.

According to yet another embodiment of the *in vitro* method for diagnosing a malignant erythroderma as defined herein, a combination of five biomarkers consisting of Tox, PLS3, Twist, KIR3DL2, and NKp46, is used.

The invention further relates to the *in vitro* method above, wherein said biomarkers are further quantified in said step a), thereby obtaining quantitative biomarker data from said subject.

In a preferred embodiment, the invention relates to said *in vitro* method, wherein said quantitative biomarker data from said subject is compared to quantitative biomarker data of reference obtained from control subjects in said step b).

These control subjects can be either healthy or diseased depending on the purposes of the method. Preferably, these control subjects are diseased subjects.

Quantifying biomarkers can be accomplished by methods well known in the art.

The biomarker quantification can be accomplished by measuring protein expression level. Alternatively or in combination, the biomarker quantification can be accomplished by measuring the mRNA expression level.

In a particular embodiment, the level of biomarker mRNA can be determined by *in vitro* assays in a biological sample, more preferably in a blood-derived sample, using methods known in the art.

The isolated mRNA can be used in hybridization or amplification assays that include, but are not limited to, Southern analyses, Northern analyses, polymerase chain reaction (PCR) analyses, and probe arrays.

A preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under conventional stringent conditions to a mRNA or genomic DNA encoding a biomarker of the present invention. Hybridization of an mRNA with the probe indicates that the biomarker under consideration is being expressed.

In one embodiment, the mRNA is immobilized on a solid surface and contacted with a probe, for example by running the isolated mRNA on an agarose gel and transferring the mRNA from the gel to a membrane, such as nitrocellulose. In an alternative assay, the probe(s) are immobilized on a solid surface and the mRNA is contacted with the probe(s), for example, in an Affymetrix gene chip array. One skilled in the art can readily adapt known mRNA detection methods for use in detecting the level of mRNA encoded by the biomarkers of the present invention. An alternative method for determining the level of mRNA biomarker in a sample involves the process of nucleic acid amplification, e.g., by quantitative RT- PCR, ligase chain reaction, self sustained sequence replication, transcriptional amplification system, Q-Beta Replicase, rolling circle replication or any other nucleic acid amplification method, followed by the detection of the amplified molecules using techniques well known to those of one skilled in the art. These detection schemes are especially useful for the detection of nucleic acid molecules if such molecules are present in very low numbers.

As used herein, amplification primers can be pairs of nucleic acid molecules that can anneal to 5" or 3" regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

PCR is a highly sensitive-and powerful method for such biological biomarkers quantification. For performing any one of the nucleic acid amplification method that is appropriate for quantifying a biomarker when performing the *in vitro* diagnosis method of the invention, a pair of primers that specifically hybridize with the target mRNA or with the target cDNA is required. A pair of primers that specifically hybridize with the target nucleic acid biological biomarker of interest may be designed by any one of the numerous methods known in the art.

In some embodiments, for each of the biological biomarkers of the invention, at least one pair of specific primers, as well as the corresponding detection nucleic acid probe, is already referenced and entirely described in the public "Quantitative PCR primer database", available on the web, especially at the following Internet address: http://lpgws.nci.nih.gov/cgi-bin/PrimerViewer.
Many specific adaptations of the PCR technique, in particular real-time or quantitative PCR (QPCR), multiplex quantitative PCR, microfluidic PCR, are known in the art for both qualitative and quantitative detections.

Illustrative embodiments of quantification biological markers using nucleic acid amplification methods are disclosed in the examples herein.

According to general embodiments of the *in vitro* diagnosis method described herein, quantifying the level of cellular protein expression may be performed for instance (i) by measuring the amount of said protein contained in a whole cell sample or (ii) by measuring the amount of said protein that is present at the cell surface, preferably at the CD4⁺ T cell surface or at the CD3⁺ T cell surface.

Measuring the amount of a protein biomarker of interest contained in a whole cell sample may be performed by Western blotting starting from the soluble fraction of a cell lysate and using an antibody directed against said protein biomarker of interest, according to methods that are well known by the one skilled in the art.

The term "antibody" as used herein designates a polypeptide that exhibit binding specificity to a specific antigen. More particularly, an antibody (or "immunoglobulin") consists of a glycoprotein comprising at least two heavy (H) chains and two light (L) chains inter-connected by disulfide bonds. Each heavy chain comprises a heavy chain variable region (or domain) (known as V_{H}) and a heavy chain constant region (known as C_{H}). Heavy chains are classified as gamma, mu, alpha, delta, or epsilon, and define the antibody's isotype as IgG, IgM, IgA, IgD, and IgE, respectively. The C_{H} region of the immunoglobulin IgG, IgD, and IgA (y, δ and α chains respectively) comprises three domains (CH1, CH2, and CH3) and a hinge region for added flexibility, while the C_{H} region of the immunoglobulin IgM and IgE contains 4 domains (CH1, CH2, CH3, and CH4). The antibodies can be polyclonal or monoclonal antibodies. For example, antibodies can be rabbit polyclonal antibodies or mouse monoclonal antibodies.

Measuring the amount of a protein biomarker of interest that is present at the cell surface may be performed by immunochemistry, either by immuno-labeling of fixed cells or by flow immuno-cytometry, according to methods that are well known by the one skilled in the art.

Thus, specific embodiments of quantifying the selected biomarkers according to the *in vitro* diagnosis method described herein encompass those wherein:
(i) the selected biomarkers are quantified by immunochemical methods, which encompass quantification of one or more protein biomarkers of interest by immuno-detection methods, for example using antibodies directed specifically against each of the said one or more protein biomarkers, according to well-known immuno-detection methods, e.g. flow cytometry, and
(ii) the selected biomarkers are quantified by gene expression analysis, which encompasses quantification of one or more biomarker mRNAs of interest, for example by performing a Real-Time PCR Taqman PCR analysis, as it is shown in the examples herein.

More particularly, "data of reference" relates to the mRNA level expression relative to the various biomarkers in CD4⁺T cells isolated from healthy human subject. Yet particularly, data of reference relating to Tox is 21.1+/-8.3. Yet particularly, data of reference relating to PLS3 is 2.4+/-2.5. Yet particularly, data of reference relating to twist is 6.6+/-8. Yet particularly, data of reference relating to KIR3DL2 is 22.5+/-20.4. Yet particularly, data of reference relating to NKp46 is 2.6+/-2.5. Even more particularly, the data of reference of the biomarker Tox is about 29,4, the data of reference of the biomarker twist is about 14.6, the data of reference of the biomarker KIRD3DL2 is about 42,9, the data of reference of the biomarker NKp46 is about 5.1 and the data of reference of PLS3 is about 4,9. All these values are expressed as 2^{ΔΔCt} mean value (A.U) +/- SEM CD4+ T cells isolated from healthy donors.

In a preferred embodiment of the *in vitro* method as defined above, said biomarkers are quantified by measuring the level of mRNA expression in said blood sample.

As shown in the examples herein, said biomarkers may be quantified by performing the well-known quantitative real-time PCR amplification technique, wherein primers specific for each of the biomarkers used among TOX, PLS3, Twist, KIR3DL2, and NKp46, are used.

By "nucleic acids of interest", it is meant herein the nucleic acid regions or segments containing the biomarkers that are indicative of malignant erythroderma. In this respect, the nucleic acids of interest may be larger than the biomarkers or they may be limited to the biomarkers.

"Probes" and "primers" are oligonucleotides that hybridize in a base-specific manner to a complementary strand of nucleic acid molecules. By "base-specific manner", it is meant that the two sequences must have a degree of nucleotide complementarity sufficient for the primer or the probe to hybridize. Accordingly, the primer or probe sequence is not required to be perfectly complementary bases or modified bases can be interspersed into the primer or probe, provided that base substitutions do not inhibit hybridization.

A probe or primer usually comprises a region of nucleic acid that hybridizes to at least about 8, preferably about 10, 12, 15, more preferably about 20, 25, 30,35, and in some case, about 40, 50, 60, 70 consecutive nucleotides of the nucleic acid template.

The primers and probes are typically at least 70% identical to the contiguous or complementary nucleic acid sequence (which is the "template"). Identity is preferably of at least 80%, 90%, 95%, and more preferably, of 98%, 99%, 99,5%, 100%.

Advantageously, the primers and probes further comprise a label, e.g., radioisotope, fluorescent compound, enzyme, or enzyme co-factor.

Another aspect of the invention is directed to a method for selecting pharmaceutical agents useful in the prevention and/or treatment of malignant erythroderma.

In some embodiments, the level of mRNA expression for each of the biomarkers tested is performed using the well-known technique of real-time PCR, then forming complexes between the double-stranded nucleic acids resulting from amplification and fluorescent molecules and then by measuring the fluorescence signal generated by the fluorescent molecules complexed with said amplified nucleic acids. The fluorescent molecules are well known by one skilled in the art.

Primers specific for each of the biomarkers mRNA consists of a routine work for the one skilled in the art. Illustratively, the one skilled in the art may use the biomarker-specific primers for each of PLS3, Twist, KIR3DL2 and NKp46 that are disclosed in the examples herein.

In some preferred embodiments, the quantification of Tox may be performed by using the pair of primers of SEQ ID N° 1 and 2
N°1: forward 5'- CTGAAGCAACTCGCAGCA-3'
N°2: reverse 5'-ACGTCAACAGGTTCACTGTAG-3'

In some preferred embodiments, quantification of PLS3 may be performed by using the pair of primers of SEQ ID N° 3 and 4,
SEQ ID N°3: forward 5'-GACTGCTTTGGCAGATCATTAAGA-3'
SEQ ID N°4: reverse 5'-AAGCAGCCAAGGCTTCATTC-3'

In some preferred embodiments, quantification of Twist may be performed by using the pair of primers of SEQ ID N° 5 and 6,
SEQ ID N°5: forward 5'-CACTGAAAGGAAAGGCTACA-3'
SEQ ID N°6: reverse 5'-GGCCAGTTTGATCCCAGTAT-3'

In some preferred embodiments, quantification of KIRDL2 may be performed by using the pair of primers of SEQ ID N° 7 and 8,
SEQ ID N°7: forward 5'-CCTGCGTGTGGTCAAACTCA-3'
SEQ ID N°8: reverse 5'-TGGGTGAAGGCCAACTACTTG-3'

In some preferred embodiments, quantification of NKp46 may be performed by using the pair of primers of SEQ ID N° 9 and 10,
SEQ ID N°9: forward 5'-CCCAGTGAGCCAGTGAAGCT-3'
SEQ ID N°10: reverse 5'-TGCAAGGCTGGTGTTCTCAA-3'

Normalization of gene expression may be performed by quantification of B2microglobulin (B2m) using the pair of primers of SEQ ID N° 11 and 12,
SEQ ID N°11: forward 5'-TGCTGTCTCCATGTTTGATGTATCT-3'
SEQ ID N°12: reverse 5'-TCTCTGCTCCCCACCTCTAAGT-3'.

In a particular embodiment of the *in vitro* diagnosis method, which is illustrated in the examples herein, quantification of the target gene expression may be performed using the comparative cycle threshold (Ct) method according to the manufacturer's instructions (Applied Biosystems). An average Ct was calculated for the duplicate reactions and normalized to B2microglobulin (ΔCt=Ct sample-Ct B2microglobulin). The obtained Cts values are expressed as arbitrary units (A.U.). Beyond referring to the examples herein, the one skilled in the art may find the appropriate teachings for performing this technique in the article of McCurdy et al. (2008, Gene Therapy and Molecular Biology, Vol.12, p. 15).

Another object of the invention is the *in vitro* method, wherein said biomarkers are quantified by measuring the expression level of cellular proteins, in particular of cell surface proteins, in said blood sample.

In some embodiments of the *in vitro* diagnosis method herein, said biomarkers are quantified by measuring the level of cellular protein expression, by western blot analysis or immunocytochemistry, notably especially for cytoplasmic biomarkers Tox, PLS3 and Twist.

In some embodiments of the *in vitro* diagnosis method herein, said biomarkers are quantified by measuring the level of cell surface or membrane protein expression of the biomarkers of interest, and notably especially for each of the selected biomarkers KIR3DL2 and NKp46, e.g. by using flow cytometry.

In another embodiment, measuring the level of cellular protein expression may be performed using suitable antibodies directed against one of the Tox, PLS3, Twist, KIR3DL2 and NKp46 proteins.

Illustratively, anti-Tox antibodies that may be used encompass the rabbit polyclonal antibody N1C1 KIAA0808 from GeneTex (ref. GTX115287), commercialized by the Company Tebu-Bio, Le Perray en Yvelines, France.

Further illustratively, anti-PLS3 antibodies that may be used encompass the rabbit polyclonal antibodies commercialized by the Company Thermo Fischer Scientific under the Reference number PA1-41262, the rabbit polyclonal antibodies commercialized by the Company Aviva Systems Biology under the reference number ARP56623-P050 and the rabbit polyclonal antibodies commercialized by the Company Biorbyt under the reference number orb6736. Anti-PLS3 antibodies that may be used also encompass the mouse monoclonal antibody commercialized by the Company "Santa Cruz Biotechnology" under the Reference number T-plastin (A-3) sc-166208.

Further illustratively, anti-Twist antibodies that may be used encompass the rabbit polyclonal antibodies commercialized by the Company Lifespan Biosciences under the reference number LS-B6741, the mouse monoclonal antibodies commercialized by the Company AbD Serotec under the reference number MCA4996Z and the mouse monoclonal antibodies Commercialized by the Company Genway under the reference number GWB-1FE920. Anti-Twist antibodies that may be used also encompass the rabbit polyclonal antibody commercialized by the Company Santa Cruz Biotechnology under the Reference number twist (H-81): sc-15393.

Still illustratively, anti-KIR3DL2 antibodies that may be used encompass the rabbit polyclonal antibodies commercialized by the Company Thermo Fischer Scientific under the reference number PA5-26224, the rabbit polyclonal antibodies commercialized by the Company Abgent under the reference number AP9215a and the rabbit monoclonal antibodies commercialized by the Company Aviva Systems under the reference number OAAB05807. Anti-KIR3DL2 antibodies that may be used also encompass the rabbit polyclonal antibody commercialized by the Company Thermo Fischer Scientific under the reference number PA5-26224, or the monoclonal mouse anti-CD158e/k (KIR3DL1/DL2) antibody commercialized by the Company Myltenyi Biotec under the Reference number 130-095-205 for PE-conjugated antibody or 130-095-199 for pure antibody.
Yet illustratively, anti-NKp46 antibodies that may be used encompass the mouse monoclonal antibodies commercialized by the Company Lifespan Biosciences under the reference number LS-B2105, the mouse monoclonal antibodies commercialized by the Company AbD Serotec under the reference number MCA4646PE and the mouse monoclonal antibodies commercialized by the Company AbD Serotec under the reference number MCA4646FT. Anti-NKp46 antibodies that may be used also encompass the mouse monoclonal antibody commercialized the Company Beckman Coulter under the Reference number PN-IM3711 for PE-conjugated antibody.

Still another object of the invention is the *in vitro* method, which further comprising a step c) of concluding to a risk and/or of diagnosing and/or of prognosing malignant erythroderma if said quantitative biomarker data from said subject is significantly higher than said quantitative biomarker data of reference.

By "significantly higher", it is meant herein that when the quantification of the biomarker's mRNA expression level is higher than the data of reference, the diagnosis of a malignant erythroderma, more preferably a CTCL-associated erythroderma can be done.

In some embodiments of the *in vitro* diagnosis method described herein, the biomarkers quantification is performed on a provided blood sample consisting of whole blood.

In some other embodiments of the *in vitro* diagnosis method described herein, the biomarkers quantification is performed on a provided blood sample selected from the group consisting of: purified blood leucocytes, peripheral blood mononuclear calls (PBMC), purified CD4⁺T cells, and purified CD3⁺T cells.

A whole blood sample as well as the purified cells samples mentioned above may be also termed "blood-derived sample" herein.

As it has been already specified above, the inventors have determined critical threshold values for each of the biomarkers among PLS3, Twist, KIR3DL2 and NKp46 allowing to reliably discriminate between SS positive individuals and SS negative individuals, when the expression level of specific combinations of at least two biomarkers among these five biomarkers are measured.

A threshold value for a specific marker among Tox, PLS3, Twist, KIR3DL2 and NKp46 may be determined by carrying out a method comprising the steps of:
a) providing (i) a collection of samples from individuals already diagnosed for being SS positive and (ii) a collection of samples from individuals diagnosed for being malignant erythroderma negative,
b) quantifying, for each sample from collection (i) provided at step a), one biomarker selected from the group consisting of Tox, PLS3, Twist, KIR3DL2 and NKp46, whereby a first collection of quantification values for said biomarker is obtained,
c) quantifying, for each sample from collection (ii) provided at step a), one marker selected from the group consisting of Tox, PLS3, Twist, KIR3DL2 and NKp46, whereby a second collection of quantification values for said biomarker is obtained,
d) calculating, from said first collection of quantification values obtained at the end of step b), the mean quantification value for said marker in SS-negative human subject,
e) calculating, from said second collection of quantification values obtained at the end of step b), the mean quantification value for said marker in SS-positive human subject,
f) calculating, for the marker tested, a threshold value that optimally discriminates between SS positive and SS negative human subject from the mean quantification values obtained at steps d) and e), respectively.

For the sake of clarity, the expression « optimally discriminates » that is used for describing step f) of the method above means that said threshold value is calculated and said value lies between (i) the mean quantification value that is obtained at step d) and the mean quantification value that is obtained at step e) and is the most discriminating between SS positive and SS negative human subject. In the same time, it shall be understood that performing an *in vitro* method of assessing the expression level of a single marker among Tox, PLS3, Twist, KIR3DL2 and NKp46, even by using the highly discriminating predetermined threshold value found for said marker, does not allow to reliably perform a diagnosis of the occurrence of SS in a human subject.

This explains why the method of determining a threshold value described above shall be performed for each marker used in the in *vitro* diagnosis method of the invention, for the purpose of performing a reliable diagnosis of a SS in an human subject.

In some embodiments, the threshold value of 95% significance is set up for each marker. According to these embodiments, when performing the *in vitro* method for predicting the occurrence of a SS described herein, any expression level value of a specific marker that is measured and that is found lower that the threshold value determined for said marker is contributive to an indication of a SS negative human subject. According to these embodiments, when performing the *in vitro* method for predicting the occurrence of a SS described herein, any expression level value of a specific marker that is measured and that is found higher that the threshold value determined for said marker is contributive to an indication of a SS positive human subject.

The inventors believe that one reason which explains the high statistical relevance of the *in vitro* diagnosis method disclosed herein, for predicting the occurrence of a SS in an individual, consists of the size of the patients cohort that have been tested, which provide highly accurate threshold values for the relevant markers and thus accurate discrimination between Sezary-positive and Sezary-negative patients.

Indeed, the threshold values that may be used when performing the *in vitro* diagnosis method disclosed herein may be expressed as arbitrary units that reflect the expression level of said marker in the sample analyzed, said expression level either consisting a protein expression level, e.g. a cell surface expression level, or a gene expression level, e.g. a mRNA expression level.

Using the expression level value of each marker of specific combinations of the biomarker Tox and one or more biomarkers among PLS3, Twist, KIR3DL2 and NKp46, a biomarker quantification value is found contributive to an indication of the occurrence of a SS when said quantification value is above the predetermined threshold value found or predetermined for said biomarker.

As it has been also already specified above, it is shown by the inventors herein that, using said threshold values for each biomarker tested, a highly reliable, i.e. highly sensitive, highly selective, and highly reproducible, prediction of the occurrence of a SS may be performed when practicing the *in vitro* diagnosis method that is described herein.

In a preferred embodiment, the *in vitro* method is for identifying human subjects at risk for developing malignant erythroderma, preferably CTCL, yet preferably SS or MF.

In a preferred embodiment the *in vitro* method is for diagnosing malignant erythroderma, preferably CTCL, yet preferably in a human subject.

In a preferred embodiment the *in vitro* method is for selecting an efficient and safe anti-malignant erythroderma therapy, preferably an anti-CTCL therapy, yet preferably an anti-SS therapy for administration to a human subject in need thereof.

In a preferred embodiment the *in vitro* method is for monitoring the effect of an anti-malignant erythroderma therapy, preferably an anti-CTCL therapy, yet preferably an anti-SS therapy administered to a human subject having malignant erythroderma, preferably CTCL, yet preferably SS.

The present invention also relates to a method for adapting an anti-malignant erythroderma treatment, preferably anti-CTCL treatment, more preferably anti-SS treatment or anti-MF treatment in a patient, wherein said method comprises the steps of:
a) performing, on at least one sample collected from said patient, and more preferably a blood-derived sample previously collected from said patient, the *in vitro* diagnosis method that is disclosed herein; and
b) adapting the anti-malignant erythroderma treatment of said patient by administering to said patient a suitable therapy.

Monitoring the influence of agents (e.g., drug compounds) on the level of expression of a biomarker selected from the group consisting of Tox, PLS3, Twist, KIR3DL2 and NKp46 can be applied for monitoring the status of malignant erythroderma in a patient with time. For example, the effectiveness of an agent to affect biomarker expression can be monitored during treatments of subjects receiving anti-malignant erythroderma treatments, preferably anti-CTCL treatments.

In a preferred embodiment, the method for monitoring the effectiveness of treatment in a human subject in need thereof, with an agent (e.g., an agonist, antagonist, peptidomimetic, protein, peptide, nucleic acid, small molecule, or other drug candidate) comprises the steps of:
(i) obtaining a pre-administration sample from a diseased subject prior to administration of the agent;
(ii) detecting the level of expression of a combination of the biomarker TOX and at least one biomarker selected from the group consisting of: Twist, PLS3, KIRD3DL2, and NKp46, thereby obtaining biomarker data from said subject, in the pre-administration sample;
(iii) obtaining one or more post-administration samples from the subject;
(iv) detecting the level of expression of the same bio markers in the post-administration samples;
(v) comparing the level of expression of said biomarkers in the pre- administration sample with the level of expression of said b i o markers in the post-administration sample(s); and
(vi) adapting the administration of the agent to the subject accordingly.

As an example of such adaptation in step vi), a worse diagnosis that is determined by assessing the expression level of the selected biomarkers during the course of treatment may indicate ineffective dosage and the desirability of increasing the dosage. Conversely, a better diagnosis that is determined by assessing the expression level of the selected biomarkers may indicate efficacious treatment and no need to change dosage.

In a preferred embodiment the *in vitro* method is for predicting the effectiveness of a therapy to treat malignant erythroderma, preferably CTCL, preferably SS in a subject in need of such treatment.

In a preferred embodiment the *in vitro* method is for selecting an efficient and safe preventive therapy for administration to a human subject at risk for developing malignant erythroderma, preferably CTCL, preferably SS or MF.

The present invention also provides a method for monitoring the effect of a preventive therapy administered to a human subject at risk for developing malignant erythroderma, preferably CTCL, preferably SS.
In a preferred embodiment, the method for monitoring the effectiveness of treatment in a human subject at risk, with an agent (e.g., an agonist, antagonist, peptidomimetic, protein, peptide, nucleic acid, small molecule, or other drug candidate) comprises the steps of:
(i) obtaining a pre-administration sample from a subject at risk prior to administration of the agent;
(ii) detecting the level of expression of a combination of the biomarker TOX and at least one biomarker selected from the group consisting of: Twist, PLS3, KIRD3DL2, and NKp46, thereby obtaining biomarker data from said subject, in the pre-administration sample;
(iii) obtaining one or more post-administration samples from the subject;
(iv) detecting the level of expression of the same bio markers in the post-administration samples;
(v) comparing the level of expression of said biomarkers in the pre- administration sample with the level of expression of said b i o markers in the post-administration sample(s); and
(vi) adapting the administration of the agent to the subject accordingly.

As an example of such adaptation in step vi), a worse diagnosis that is determined by assessing the expression level of the selected biomarkers during the course of treatment may indicate ineffective dosage and the desirability of increasing the dosage. Conversely, a better diagnosis that is determined by assessing the expression level of the selected biomarkers may indicate efficacious preventive therapy.

The present invention also relates to a method for adapting a preventive anti-malignant erythroderma treatment, preferably anti-CTCL treatment, more preferably anti-SS treatment or anti-MF treatment, in a human subject at risk, wherein said method comprises the steps of:
a) performing, on at least one sample collected from said human subject, and most preferably a blood-derived sample previously collected from said human subject, the *in vitro* diagnosis method that is disclosed herein; and
b) adapting the preventive anti-malignant erythroderma treatment of said human subject by administering to said human subject a suitable preventive therapy.

Thus, in a preferred embodiment, the *in vitro* method is for predicting the effectiveness of a therapy to prevent malignant erythroderma, preferably CTCL, preferably SS, in a human subject at risk.

In a more preferred embodiment of the *in vitro* method, said biomarkers in said step a) are selected from the group consisting of:
- TOX and Twist;
- TOX and PLS3;
- TOX and KIR3DL2;
- TOX and NKp46;
- TOX, twist, and PLS3;
- TOX, twist, and KIR3DL2;
- TOX, twist, and NKp46;
- TOX, twist, PLS3, and KIR3DL2;
- TOX, twist, PLS3, and NKp46; and
- TOX, twist, PLS3, KIR3DL2, and NKp46.

Another aspect of the invention provides a test kit for use in an *in vitro* method, to make risk assessment and/or diagnosis and/or prognosis of malignant erythroderma, preferably a CTCL in a human subject, wherein said kit comprises appropriate means for:
a) assessing the presence of, and preferably quantifying, in a blood sample of a human subject, the biomarker TOX and at least one biomarker selected from the group consisting of: Twist, PLS3, KIRD3DL2, and NKp46; and
b) Comparing the biomarker data obtained in a) from said subject to biomarker data of reference obtained from healthy and/or diseased subjects, to make risk assessment and/or diagnosis and/or prognosis of malignant erythroderma, preferably CTCL, in said human subject.

The terms "test kit" and "kit" are synonymous and may be used interchangeably.

In the context of the present invention when reference is made to test kit, the terms "appropriate means" refer to any technical means useful for achieving the indicated purpose. As non-limiting examples of such appropriate means, one can cite reagents and/or materials and/or protocols and/or instructions and/or software, etc. all the kits of the present invention may comprise appropriate packaging and instructions for use methods herein disclosed. The kits may further comprise appropriate buffer(s) and polymerase such as thermostable polymerases, for example Taq polymerase. Such Kits may also comprise control primer and/or probes.

The kit can comprise a plurality of reagents, each of which is capable of binding specifically with a biomarker nucleic acid or a biomarker protein among Tox and at least one other biomarker selected from the group of: PLS3, Twist, KIR3DL2 and NKp46.

Suitable reagent for binding with a biomarker protein include antibodies, antibody derivatives, antibody fragment, and the like.

Suitable reagents for binding with a biomarker nucleic acid (e.g. a genomic DNA, an mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

According to a preferred embodiments, the kits of the invention may comprise at least:
a) One isolated PCR primer pair consisting of a forward primer and a reverse primer, for specifically amplifying nucleic acids of interest; and/or
b) One isolated primer for specifically extending nucleic acid of interest; and/or
c) One isolated nucleic acid probe specifically binding to nucleic acids of interests and/or
d) One isolated antibody specifically binding protein(s) encoded by nucleic acid(s) of interest; and/or
e) One microarray or multiwall plate comprising at least one of a) to d) above.

A further object of this invention consists of a kit for the diagnosis of a malignant erythroderma, preferably CTCL, more preferably a SS or a MF, said kit comprising means for quantifying in a blood sample of a human subject, combinations of at least two biomarkers, namely the biomarker TOX and at least one biomarker selected from the group consisting of: Twist, PLS3, KIRD3DL2, and NKp46.

More precisely, this invention relates to a kit for the diagnosis of a malignant erythroderma, preferably CTCL, more preferably a SS or a MF, said kit comprising means for quantifying in a blood sample of a human subject the level of expression of a combination of at least two biomarkers, namely the biomarker TOX and at least one biomarker selected from the group consisting of: Twist, PLS3, KIRD3DL2, and NKp46.

The kit of the invention may optionally comprise additional components useful for performing the methods of the invention. By way of example, the kit may comprise fluids (e.g. SSC buffer) suitable for annealing complementary nucleic acids or for binding an antibody with a protein with which it specifically binds, one or more sample compartments, an instructional material which describes performance of the *in vitro* diagnosis method of the invention, and the like. Kits comprising antibodies.

In another embodiment, a kit according to the invention comprises one or a combination or a set of antibodies, each kind of antibodies being directed specifically against the Tox, PLS3, Twist, KIR3DL2 and NKp46 nucleic biomarkers of the invention.

In one embodiment, said kit comprises a combination or a set of antibodies comprising at least two distinct antibodies, each antibody being selected from the group consisting of antibodies directed against the Tox, PLS3, Twist, KIR3DL2 and NKp46 protein biomarkers, and said kit comprises a combination of at least two antibodies, anti-Tox, and at least one antibody selected from the group of antibody consisting of: anti-PLS3, anti-Twist, anti-KIR3DL2 and anti-NKp46.

In some embodiments, said kit comprises a combination or a set of antibodies selected from the group of antibodies directed against protein biomarkers sets, said group of antibodies consisting of:
- anti-TOX and anti-Twist;
- anti-TOX and anti-PLS3;
- anti-TOX and anti-KIR3DL2;
- anti-TOX and anti-NKp46;
- anti-TOX, anti-twist, and anti-PLS3;
- anti-TOX, anti-twist, and anti-KIR3DL2;
- anti-TOX, anti-twist, and anti-NKp46;
- anti-TOX, anti-twist, anti-PLS3, and anti-KIR3DL2;
- anti-TOX, anti-twist, anti-PLS3, and anti-NKp46; and
- anti-TOX, anti-twist, anti-PLS3, anti-KIR3DL2, and anti-NKp46.

In still another embodiment, said kit comprises a specific set or a specific combination of nucleic acid primers, or pairs of primers, each primer or pair of primer hybridizing with each of PLS3, Twist, KIR3DL2 and NKp46 nucleic acid biomarkers, which includes PLS3, Twist, KIR3DL2 and NKp46 mRNAs and PLS3, Twist, KIR3DL2 and NKp46 cDNAs. A primer kit according to the invention may comprise at least two kinds of pair or primers, each kind of pair of primers hybridizing specifically with the biomarker Tox and at least one biomarker selected from the group consisting of PLS3, Twist, KIR3DL2 and NKp46.

In addition, the present invention concerns the use of a test kit as described above for stratifying humans subjects into particular groups of biomarker expression (expression of 2, 3, 4 or 5 markers).

Advantageously, this test kits is further used for selecting at least one human subject from at least one group for inclusion in a clinical trial to assess the efficacy of a therapy for treating and/or preventing malignant erythroderma, preferably CTCL, yet preferably SS or MF.

The present invention is further illustrated by, without in any way being limited to, the examples below.

### FIGURE:

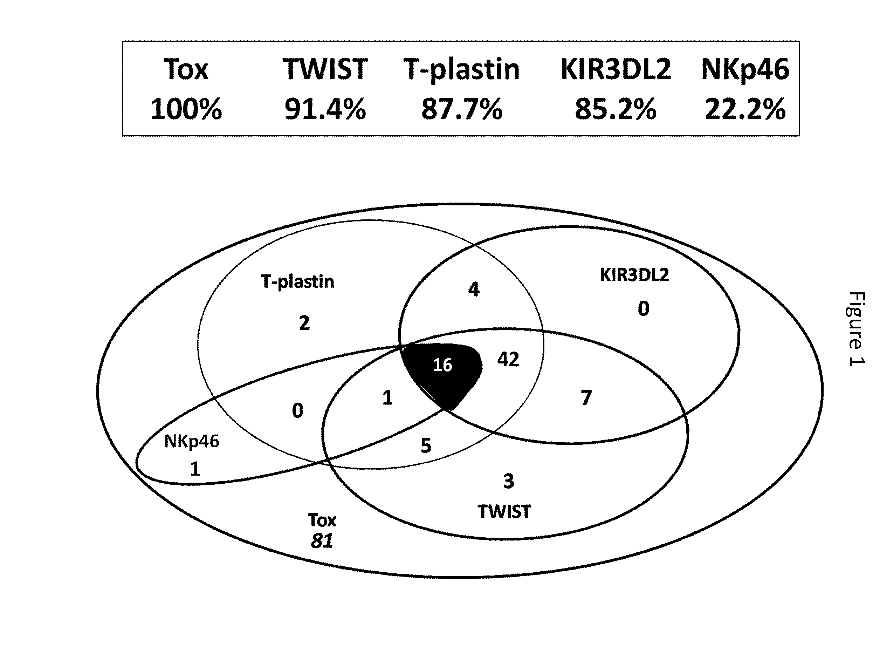

Figure 1: Schematic representation of percentages of positive patients formRNA expression of PLS3, Twist, KIR3DL2, NKp46 and Tox from a well-defined unicentric cohort of 81 patients.

### EXAMPLES :

### I- Materials and methods

### I-1 Patients

Peripheral blood samples from patients affected by Sézary syndrome or with Mycosis Fungoides were collected. Diagnosis of SS and MF was based on clinical, histologic and biological criteria [*Willemze et al., 2005; Olsen et al. 2011*]*.* According to the revised staging and classification criteria for MF and SS including TNMB (tumor-node-metastasis-blood) status, patients with SS were characterized by a staging II, III or IV and patients with MF by a staging I or II. Within the following 24h of blood collection, peripheral blood mononuclear cells (PBMCs) were isolated by Ficoll-Hypaque centrifugation and peripheral blood lymphocytes (PBLs) isolated from PBMCs by elimination of monocytes by adhesion for 2 hours.

For comparative analysis, blood samples were obtained from healthy controls from "Etablissement Francais du Sang" (EFS).

### 1-2 RNA isolation and cDNA synthesis

RNA was isolated from whole PBMCs or isolated CD4⁺ T cells using RNeasy Mini Kit according to the manufacturer's instructions with one step DNAse I (Qiagen). For cDNA synthesis, total RNA (1µg) was used for Reverse Transcription (RT) with ThermoScript reverse transcriptase (Invitrogen) using oligo-dT primers.

### 1-3 Multiplex and Quantitative Real Time-PCR analysis

The resulting first-strand cDNA was assayed by standard multiplex PCR using the QIAGEN^{®} Multiplex PCR Kit according to the manual instruction and, by quantitative real-time PCR (qRT-PCR) using SYBR Green PCR Core Reagents (ABI PRISM 7300 Real-time PCR System, Applied Biosystems) according to the manufacturer's instructions. Details concerning primers and qRT-PCR conditions are described above.

### 1-4 Immunoblot analysis

Peripheral T cells (10x10⁶) were lysed for 1h at 4°C in RIPA Lysis Buffer (Rockland Immunochemicals) supplemented with Protease Inhibitor Cocktail (Sigma). After centrifugation (14,000 rpm, 15min, 4°C), whole-cell protein extracts (25-50µg) were loaded onto 10% polyacrylamide gels containing SDS, subjected to electrophoresis, and transferred to nitrocellulose membrane. The blot was blocked with 5% nonfat milk for 1h and incubated overnight at 4°C with specific antibody as detailed above (for example: anti-PLS3, 1/500; Santa-Cruz). They were subsequentially exposed to anti-mouse (1/2,500) antibody coupled to horseradish peroxidase (Amersham Pharmacia Biotech) for 1 h. An enhanced chemiluminescent system (Amersham Pharmacia Biotech) was used for detection. Equal protein loading was confirmed by the use of monoclonal GAPDH or B-actin (1/4,000, Sigma). Densitometry was done using Imager FX System and analyzed using ImageJ software.

### 1-5 Flow cytometry analysis

T Cell immunophenotype was assessed by staining with specific association of APC-CD4, PerCP-CD3, Fitc-CD8, and specific biomarker antibodies, including CD158k/KIR3DL2-PE or NKp46-PE. Antibody isotypes were selected to match these specific antibodies. For membrane staining, cells were incubated with optimal concentrations of antibodies at in the staining buffer (0.5% BSA in PBS 1X buffer) for 20min at 4°C in the dark and then fixed with 1% formaldehyde. For intracellular staining, cells were fixed for 1h at 4°C in PBS containing 0.45% formaldehyde before permeabilized for 15 min at 37°C in PBS containing 0.2% Tween20, washed twice, and incubated with Tox, Twist or T-plastin specific antibodies at optimal concentrations in PBS for 30min at 4°C in the dark and then washed in PBS+2% FCS. Cells were next incubated with antibodies for membrane staining and finally fixed with 1% formaldehyde. Surface and intracellular expression was quantified using a FACSCalibur^{®} flow cytometer (BD Biosciences). Unstained cells or cells stained with isotype-matched antibodies were used to subtract nonspecific signals. Data were analyzed with Kaluza^{®} software (BD Biosciences).

### II- Results

Table 1 below and appended Figure 1 show quantitative gene expression of the 5 biomarkers T-plastin, TWIST, Kir3DL2, NKp46, Tox in CD4⁺ T cells.

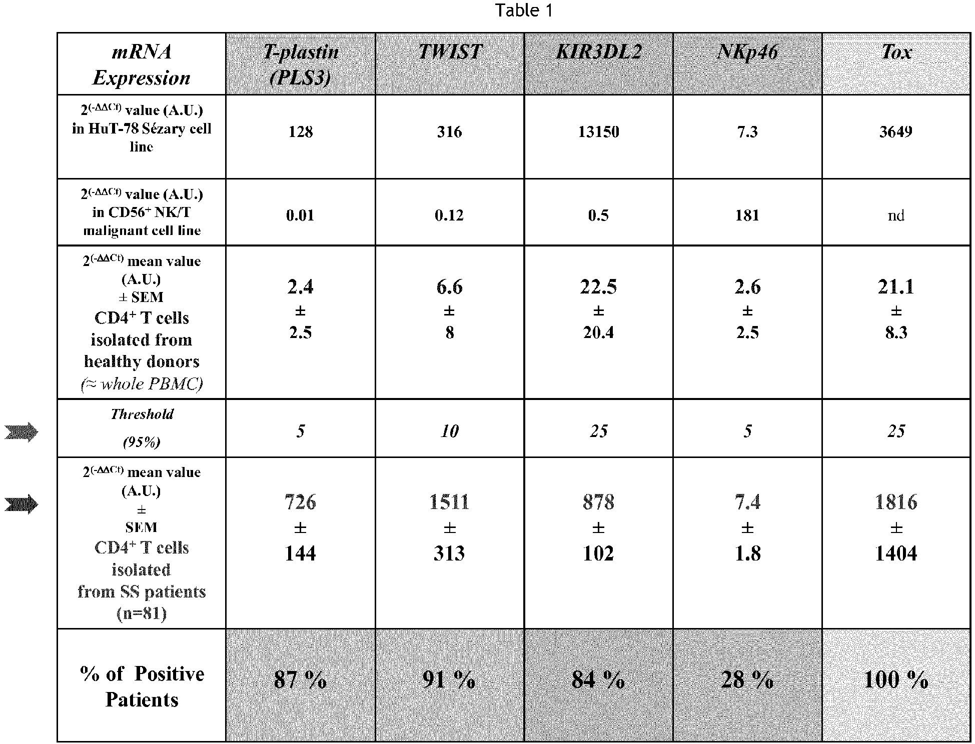

The results clearly demonstrate the undisputable interest and advantages of the present invention.

### REFERENCES

Michel et al. Use of PLS3, Twist, CD158k/KIR3DL2, and NKp46 gene expression combination for reliable Sézary syndrome diagnosis., 2013
Zhang et al., Molecular biomarkers of early-stage Mycosis Fungoides,2012
McCurdy et al., Gene Therapy and Molecular Biology, Vol.12, p.15, 2008
Willemze R, Jaffe ES, Burg G, et al. WHO-EORTC classification for cutaneous lymphomas. Blood. 2005;105(10):3768-3785*.*
Olsen EA, Whittaker S, Kim YH, et al. Clinical end points and response criteria in mycosis fungoides and sezary syndrome: a consensus statement of the international society for cutaneous lymphomas, the United States cutaneous lymphoma consortium, and the cutaneous lymphoma task force of the European organisation for research and treatment of cancer. J Clin Oncol. 2011;29(18):2598-2607

## Claims

1. An *in vitro* method for risk assessment and/or diagnosis and/or prognosis of malignant erythroderma, preferably cutaneous t-cell lymphoma (CTCL), in a human subject, comprising a step of:
a) assessing the presence, in a blood sample of said subject, of the biomarker TOX and at least one biomarker selected from the group consisting of: Twist, PLS3, KIRD3DL2, and NKp46, thereby obtaining biomarker data from said subject; and
b) comparing the thus obtained biomarker data from said subject to biomarker data of reference obtained from healthy and/or diseased subjects, to make risk assessment and/or diagnosis and/or prognosis of malignant erythroderma, preferably CTCL, in said human subject.

2. The method according to claim 1, wherein said biomarkers are further quantified in said step a), thereby obtaining quantitative biomarker data from said subject.

3. The method according to claim 2, wherein said quantitative biomarker data from said subject is compared to quantitative biomarker data of reference obtained from healthy and/or diseased subjects in said step b).

4. The *in vitro* method according to claim 2 or 3, wherein said biomarkers are quantified by measuring the level of mRNA expression in said blood sample.

5. The *in vitro* method according to claim 2 or 3, wherein said biomarkers are quantified by measuring the expression level of cellular proteins, in particular of cell surface proteins, in said blood sample.

6. The *in vitro* method according to any one of claims 3 to 4, further comprising a step c) of concluding to a risk and/or of diagnosing and/or of prognosing malignant erythroderma if said quantitative biomarker data from said subject is significantly higher than said quantitative biomarker data of reference.

7. The *in vitro* method according to any one of claims 1 to 6, wherein said blood sample is a whole blood sample.

8. The *in vitro* method according to any one of claims 1 to 7, wherein said method is for diagnosing malignant erythroderma, preferably CTCL, in a human subject.

9. The *in vitro* method according to any one of claims 1 to 7, wherein said method is for selecting an efficient and safe anti-malignant erythroderma therapy, preferably an anti-CTCL therapy, for administration to a human subject in need thereof.

10. The *in vitro* method according to any one of claims 1 to 7, wherein said method is for monitoring the effect of an anti-malignant erythroderma therapy, preferably an anti-CTCL therapy, administered to a human subject having malignant erythroderma, preferably CTCL.

11. The *in vitro* method according to any one of claims 1 to 7, wherein said method is for predicting the effectiveness of a therapy to treat malignant erythroderma, preferably CTCL, in a subject in need of such treatment.

12. The *in vitro* method according to any one of claims 1 to 7, wherein said method is for selecting an efficient and safe preventive therapy for administration to a human subject at risk for developing malignant erythroderma, preferably CTCL.

13. The *in vitro* method according to any one of claims 1 to 7, wherein said method is for monitoring the effect of a preventive therapy administered to a human subject at risk for developing malignant erythroderma, preferably CTCL.

14. The *in vitro* method according to any one of claims 1 to 7, wherein said method is for predicting the effectiveness of a therapy to prevent malignant erythroderma, preferably CTCL, in a human subject at risk.

15. A test kit for use in an *in vitro* method according to any one of claims 1 to 14, comprising appropriate means for:
a) assessing the presence of, and preferably quantifying, in a blood sample of a human subject, the biomarker TOX and at least one biomarker selected from the group consisting of: Twist, PLS3, KIRD3DL2, and NKp46; and
b) Comparing the biomarker data obtained in a) from said subject to biomarker data of reference obtained from healthy and/or diseased subjects, to make risk assessment and/or diagnosis and/or prognosis of malignant erythroderma, preferably CTCL, in said human subject.


SEQUENCE LISTING

<110>  INSTITUT NATIONAL DE LA SANTE ET DE LA RECHERCHE MEDICALE (INSERM)
       UNIVERSITE PARIS DIDEROT PARIS 7
       CENTRE NATIONAL DE LA RECHERCHE SCIENTIFIQUE (CNRS)
       ASSISTANCE PUBLIQUE - HOPITAUX DE PARIS

<120>  USE OF A NOVEL COMBINATION OF BLOOD BIOMARKERS FOR MALIGNANT
       ERYTHRODERMA

<130>  71669D34420

<160>  12

<170>  PatentIn version 3.5

<210>  1
<211>  18
<212>  DNA
<213>  Artificial

<220>
<223>  primers TOX forward

<400>  1
ctgaagcaac tcgcagca                                                    18


<210>  2
<211>  21
<212>  DNA
<213>  Artificial

<220>
<223>  primers TOX reverse

<400>  2
acgtcaacag gttcactgta g                                                21


<210>  3
<211>  24
<212>  DNA
<213>  Artificial

<220>
<223>  primers PLS3 forward

<400>  3
gactgctttg gcagatcatt aaga                                             24


<210>  4
<211>  20
<212>  DNA
<213>  Artificial

<220>
<223>  primers PLS3 reverse

<400>  4
aagcagccaa ggcttcattc                                                  20

<210>  5
<211>  20
<212>  DNA
<213>  Artificial

<220>
<223>  primers Twist forward

<400>  5
cactgaaagg aaaggctaca                                                   20


<210>  6
<211>  20
<212>  DNA
<213>  Artificial

<220>
<223>  primers Twist reverse

<400>  6
ggccagtttg atcccagtat                                                   20


<210>  7
<211>  20
<212>  DNA
<213>  Artificial

<220>
<223>  primers KIRDL2 forward

<400>  7
cctgcgtgtg gtcaaactca                                                   20


<210>  8
<211>  21
<212>  DNA
<213>  Artificial

<220>
<223>  primers KIRDL2 reverse

<400>  8
tgggtgaagg ccaactactt g                                                 21


<210>  9
<211>  20
<212>  DNA
<213>  Artificial

<220>
<223>  primers NKp46 forward

<400>  9
cccagtgagc cagtgaagct                                                   20


<210>  10
<211>  20

<212>  DNA
<213>  Artificial

<220>
<223>  primers NKp46 reverse

<400>  10
tgcaaggctg gtgttctcaa                                                    20


<210>  11
<211>  25
<212>  DNA
<213>  Artificial

<220>
<223>  primers ?2microglobulin (?2m) forward

<400>  11
tgctgtctcc atgtttgatg tatct                                              25


<210>  12
<211>  22
<212>  DNA
<213>  Artificial

<220>
<223>  primers ?2microglobulin (?2m) reverse

<400>  12
tctctgctcc ccacctctaa gt                                                 22